# EUROPEAN PATENT SPECIFICATION

(11) **EP 2 145 547 B1**
(45) Date of publication and mention of the grant of the patent: **27.06.2012**
(21) Application number: 08160078.5
(22) Date of filing: 10.07.2008
(51) Int. Cl.: A23L 1/22, C12N 9/02, A23F 5/46

(54) **Increased stability of flavor compounds**
Erhöhte Stabilität von Geschmackskomponenten
Stabilité améliorée de composés aromatiques

(43) Date of publication of application: 20.01.2010
(73) Proprietor: Kraft Foods R & D, Inc., Northfield, IL 60093 (US)
(72) Inventor: Silanes Kenny, Javier, B138HR Birmingham (GB); Degenhardt, Andreas, 37603, Holzminden (DE)
(74) Representative: HOFFMANN EITLE

(56) References cited:
- EP-A- 1 122 303
- US-A- 4 053 644
- US-A- 4 632 905
- US-A- 5 547 690
- SHIPE W F ET AL: "ENZYMATIC MODIFICATION OF MILK FLAVOR" JOURNAL OF DAIRY SCIENCE, vol. 58, no. 8, 1975, pages 1123-1126, XP009111169 ISSN: 0022-0302

## Description

### Technical Field

The present invention relates to a method for increasing the stability of thiol-containing flavors, in particular food flavor and aroma. The invention pertains to the treatment of instable aroma-relevant thiols which are found in coffee, tea, cacao, chocolate, cheese, wine, beer and others. The method comprises a step of contacting or admixing the thiol-containing flavor, or a composition containing said flavor with an enzyme catalyzing the formation of aroma-active disulfides. The invention further relates to a flavor-stabilized product obtainable by the method of the present invention and to a packaged or encapsulated product wherein an enzyme catalyzing the formation of disulfides has been introduced.

### Background of the Invention

Important aroma components of flavors are thiol-containing compounds. In particular, these flavor compounds are widely contained in foods giving off a roasted or grilled flavor during the cooking or roasting of a variety of foods, such as vegetables, eggs, meat, coffee, tea, cacao, chocolate, peanuts, cheese, as well as during the preparation of beverages such as wine and beer.

These volatile thiol-containing compounds are generally known to be unstable and may either be lost by evaporation or by interaction and reaction with other compounds present in the composition.

For examples, the characteristic aroma of freshly roasted coffee is the result of numerous thiol-containing volatile compounds which are predominantly formed during the roasting process. However, the specific coffee flavor quickly degrades, and moreover generates an unpleasant bitter aroma. This staling of roasted coffee was considered as an inevitable process attributed to a loss of volatile thiol-containing compounds in shelf-life due to evaporation, undesired reaction products and due to interaction with other coffee compounds including melanoidins. Thus, efforts have been made in the prior art in order to preserve the desirable aroma compounds and to reduce the undesirable components.

A process for recovery of flavor substances is the addition or incorporation of aroma-providing compounds, such as sulfur containing alkanes or sulfur containing ketones (EP 1 525 807) which replace or reinforce flavors or aromas lost during the preparation and storage of food or beverages. Alternatively, a precursor mixture (US 6,358,549) comprising polysulfide and a compound having a sulfhydryl group can be added to a food composition which generates an aromatic note due to the formation of thiols upon heating.

JP 08/196212 discloses the addition of sulfites in a blended additive comprising a catalase with glutathione, a sulfuric acid salt, cystein and antioxidant to maintain the characteristic aroma of coffee. Generally, sulfites and other antioxidants may react with oxidizing species and thus prevent the oxidation of the instable flavoring compounds. Alternatively, these antioxidants may also be incorporated in the packaging of food (US 4,041,209) by use of a structural multiple-ply wall filled with a sulfite preventing the penetration of oxygen into the packaging container.

WO 2004/028261 and WO 02/087360 relate to the addition of an aroma-improving or stabilizing agent comprising nucleophiles containing sulfur or nitrogen and being able to react with, complex or scavenge undesirable compounds which promote the degradation of other volatile desirable flavor compounds. Said stabilizing or aroma-improving agent can subsequently be removed from the food or beverage product.

WO 2006/018074 describes a treatment of aqueous coffee extract with polyvinylpyrrolidone which removes preferably more than 15% of the coffee solids leading to a removal of non-volatile compounds such as melanoidines which are suspected to induce aroma degradation.

Further, it is known that roasted coffee particles can be coated with a liquid coffee extract thereby forming a roasted coffee granulate having an aroma-protective coating (EP 0 646 319) or the roasted coffee beans can be furnish with a shellac coating film having gas barrier properties (JP 63 146 753).

An enzyme-catalyzed antioxidant system for beverages is described in US 6,093,436 disclosing a combination of glucose oxidase, a glucose oxidase substrate and an inorganic oxygen scavenger. This composition serves as an antioxidant in small amounts reducing the oxygen content of preferably coffee beverages.

However, none of the above mentioned methods for increasing the stability of flavors and aromas is selectively directed to the instable thiol-containing compounds which are predominately responsible for the characteristic flavor and aroma of a given food. Therefore, the commonly used methods are accompanied by the generation of unwanted side products within the complex flavor compound mixture.

### Summary of the Invention

It has been surprisingly found that the enzymatic conversion of reactive thiol groups (-SH) into disulfide groups (-S-S-) results in an enhanced stability of flavoring compounds as compared to the monosulfides and thereby having a comparable aroma and flavor impact due to the generation of aroma-active disulfides with excellent sensorial properties.

In particular, it has been found that the enzymatic conversion of unstable thiol-containing flavor compounds into disulfides results in an increased flavor or aroma stability of the composition. The present invention, prevents the decrease of flavoring compounds by evaporation, inhibits unwanted side reactions and thus, preserves the characteristic aroma of a food, such as coffee, tea, cacao, chocolate, cheese, wine, beer and others during storage or processing. The treatment method of the present invention provides aroma-active disulfides having increased stability, and being kept in the flavor composition as flavoring agents.
(1) Specifically, the invention provides a method for increasing the stability of thiol-containing flavor or aroma compounds comprising a step of contacting or admixing the thiol-containing flavor or aroma with an enzyme catalyzing the formation of disulfides and bringing said mixture into contact with oxygen.
(2) The method of item (1) may further comprise a step of agitating the mixture, or
(3) the method of items (1) and (2) may comprising a step of supplementing a product with flavors prior to, during and/or after the step of contacting or admixing the thiol-containing flavor or aroma with an enzyme catalyzing the formation of disulfides and bringing said mixtures in contact with oxygen.
(4) In particular, in the method of any of items (1)-(3) above, the thiol-containing flavor or aroma may be a food selected from coffee, coffee mixes, liquid concentrates thereof, tea, cacao, chocolate, peanuts, cheese, cheese flavor blocks, wine and beer.
(5) The enzyme catalyzing the formation of disulfides in any of the items (1)-(4) above, is a sulfhydryl oxidase from yeast.
(6) In particular, the sulfhydryl oxidase of item (5) above is sulfhydryl oxidase Ervlp.
(7) The enzyme used in the method of any of items (1)-(6), may be immobilized onto or within an insoluble matrix.
(8) The oxygen used in the method of any of items (1) - (7), is brought into contact with the mixture comprising the enzyme catalyzing the formation of disulfides and the thiol-containing flavor compound by bubbling or by injecting oxygen there through.
(9) The molar ratio of sulfhydryl oxidase to thiol groups used in the method of any of items (5) - (8) is from 1:2000-2000:1, and
(10) preferably, the ratio of sulfhydryl oxidase to thiol groups is 1:1.
(11) The catalyzed formation of disulfides of any of the items above is performed in a time range of 5 minutes to 12 hours, and
(12) preferably, in the time range of from 4-6 hours.
(13) The invention further relates to a product obtainable by the method according to any of items (1)-(12), and
(14) to a packaged or encapsulated product, wherein an enzyme catalyzing the formation of disulfides has been introduced.

### Description of the Invention

The present invention is directed to the treatment of reactive thiol groups (-SH) found in thiol-containing flavor compounds by a highly selective enzymatic conversion into aroma-active disulides of said flavor compounds using sulfhydryl oxidase.

This conversion omits the reactivity of thiols for reactions with other coffee components including melanoidins and which are responsible for the staling process of coffee. The resulting dimeric disulfide-containing flavor compounds display an enhanced time stability over the shelf life as compared to the monomeric thiols. The disulfides are kept within the product as a flavor compound and have a similar sensorial threshold as compared to the monomeric thiol-containing flavor compounds but have a milder aroma quality with some fresh notes.

The inventive use of an enzyme as an aroma-stabilizing agent instead of the commonly used agents and methods has many advantages. The specificity of the enzyme-catalysed reaction eliminates problems caused by the use of general antioxidants affecting the food composition as a whole. In particular, antioxidants may interfere with the formation of valuable flavor-active substances and react with other food components containing redox-active functional groups. Moreover, the inventive use of an enzyme results in milder reaction conditions which is preferable, particularly in food compositions.

Furthermore, the present invention relates to an aroma-stabilized product obtainable by the present method and to an encapsulated or packaged product wherein an enzyme catalyzing the formation of disulfides has been introduced.

In the following certain aspects and embodiments of the invention are described in more detail.

### Thiol-containing flavor compound

The thiol-containing flavor compounds treated with the method of the present invention are particularly present in foods and beverages such as coffee, tea, cacao, chocolate, cheese, wine, beer and others. Without being bound to the specific embodiments, examples of these thiol-containing flavor compounds found in such foods include the following:

**Table 1: Examples of thiol-containing flavour compounds**

| **Compound** | **Appr.** | **Constitution** | **Flavor** |
|---|---|---|---|
| methanethiol | MT | H₃C-SH | putrid |
| 2-furfurylthiol | FFT | 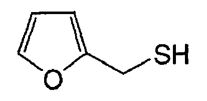 | roasty |
| 3-methyl-2-buten-1-thiol | MBT | 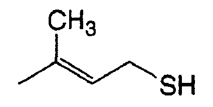 | sulfur |
| 3-mercapto-2-methyl-propanol | MMPOH | 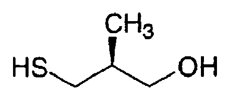 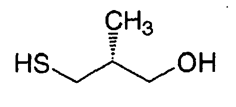 | sweat |
| 3-mercapto-3-methyl-butanol | MMB | 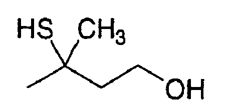 | spicy |
| 3-mercapto-3-methylbutyl-formate | MMBF | 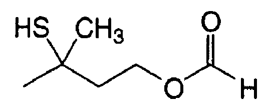 | roasty |
| 3-mercapto-3-methylbutyl-acetat | MMBA | 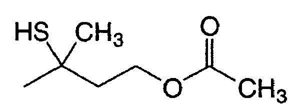 | roasty |
| 4-mercapto-4-methyl-2-pentanone | MMP | 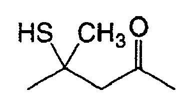 | meaty |
| 4-mercapto-4-methyl-pentane-2-ol | MMPOH | 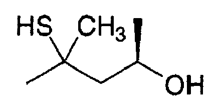 | flower lemon |
| | | 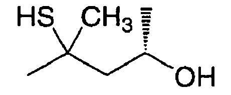 | |
| 3-mercapto-hexane-1-ol | MHOH | 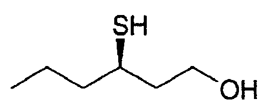 | sulfur |
| | | 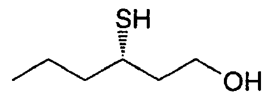 | |
| 3-mercapto-hexyl-acetat | MHA | 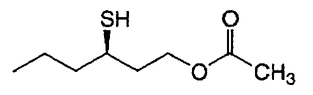 | box tree |
| | | 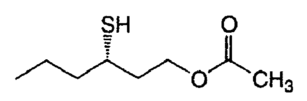 | |

### Enzyme Catalyzing the Formation of Disulfides

The enzyme catalysing the formation of disulfides as used in the present invention is generally a sulfhydryl oxidase (SOX). SOX catalyses the conversion of thiol groups into their corresponding disulfides according to the following reaction:

2 RSH + O₂ → RSSR + H₂O₂

For example, the sulfhydryl oxidase Erv1p catalyzes the reaction of 2-furfuryl thiol (FFT) into 2,2-dithiodimethylendifuran (dimeric FFT; Di-FFT) according to the following equation:

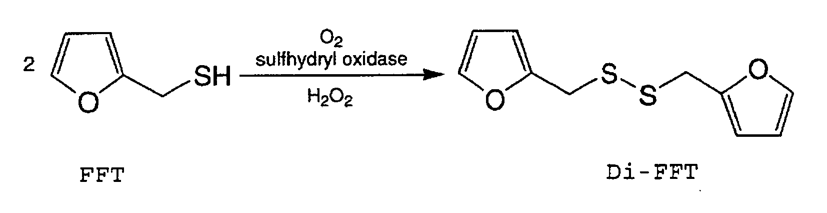

Microbial sources which generate sufficient quantities of sulfhydryl oxidase are potential sources for the isolation and production of large scale quantities thereof. The isolated sulfhydryl oxidase can be generally purified by conventional precipitation and chromatographic methods.

All experiments were carried out using the sulfhydryl oxidase Erv1p (EC 1.8.3.2 - Cat. No. E-5) by X-Zyme GmbH, Merowingerplatz 1A, 40225 Düsseldorf, Germany. This enzyme is applied to crosslinking, coating or labelling of free thiol groups, inactivation of unwanted thiol components and stabilization of the protein matric of bakery products. Erv1p is known to be active on a broad spectrum of substrates containing free thiol groups. Sulfhydryl oxidase Erv1p is made from yeast (Saccharomyces cerevisae) and is a dimeric FAD-dependent protein having a molecular weight of about 24,7 kDa per subunit.

Specific advantages of this enzyme are high thermo-stability, oxygen resistance and very favorable energetic properties. Oxygen is the only necessary substrate for the enzymatic oxidation of thiol groups into disulfides.

### Description of Figures

Figure 1: Decrease in 2-furfuryl thiol (FFT) given in percent [%] over time [h], wherein an excess of FFT (40.000 µg/kg) has been added to sulfhydryl oxidase Erv1p.
Figure 2: Generation of dimeric 2-furfuryl thiol (Di-FFT) given in percent [%] over time [h], wherein an excess of FFT (40.000 µg/kg) has been added to sulfhydryl oxidase Erv1p.
Figure 3: Stability of dimeric 2-furfuryl thiol (Di-FFT) in the presence of melanoidines compared to Methyl-2-methyl-3-furfuryldisulfid and 2-furfuryl thiol (FFT) given in percent [%] over time [h] (Example 3).
Figure 4: Enzyme treated and untreated aroma extract from roasted coffee obtained by steam evaporation. The figure shows relative concentrations of the flavor compounds obtained by GC-MS measurement.
Figure 5: GC chromatogram showing analysis of a Furfuryl model system treated with sulfhydryl oxidase Erv1p (light gray) and untreated comparative example (dark gray).

### Example 1

### Preparation of Aroma Extract (1)

Ground roast coffee of a particle size of at most approximately 1.8 mm which has been moistened to a water content of approximately 50 wt.-% relative to the ground dry roast coffee is treated in a percolator with saturated steam at a pressure of approximately 0.5 bar and a temperature of approximately 100°C for approximately 5 minutes. The steam loaded with coffee constituents is condensed at a temperature of approximately 5 °C to a condensate quantity of approximately 5 wt.-% relative to the quantity of dry roast coffee used.

### Isolation of melanoidines

The melanoidins are isolated from a coffee brew by ultrafiltration using the following steps: (a) Separation of the coffee brew in to different fractions by ultrafiltration using a molecular weight cut off of 30 kDa; (b) the remanent (> 30 kDa), i.e. isolated melanoidins, are freeze dried and used for reaction with FFT; (c) the filtrate (< 30 kDa), i.e. coffee compounds, are discarded.

### Preparation of Sulfhydryl Oxidase Solution (2)

5 mg of sulfhydryl oxidase Erv1p from yeast (X-Zyme GmbH, Merowingerplatz 1A, 40225 Düsseldorf, Germany) are dissolved in 10 mL Mcllvaine buffer (0.1 mM) at pH 7.5.

### Preparation of Furfurylmercaptane Solution (3)

A solution of 2-furfuryl thiol (Natural Advantage, Oakdale, LA, USA) is prepared in methanol (Merck, Darmstadt, Germany) having a concentration of 0.06 µg/µL.

### Preparation of a flavor-stabilized aroma extract (4)

The following quantities are used:

**Table 2: Composition for producing a flavor-stabilized aroma extract (Example 1).**

| **Component** | **Quantity** |
|---|---|
| Aroma Extract (1) | 5 mL |
| Sulfhydryl Oxidase Solution (2) | 5 mg (0.045 mmol protein) |
| Furfuryl Thiol Solution (3) | 150 µl (0.78 µmol) |
| Oxygen | 1 bubble/s |

Into a 50 mL flask 5 mL of the aroma extract (1) is transferred and 2 mL Sulfhydryl Oxidase Solution (2) is added. In order to restore original levels of furfuryl thiol in the Aroma Extract (1), 150 mL of furfuryl thiol Solution (3) are added. Subsequently, pure oxygen is bubbled through the aroma extract mixture and incubated at 40°C for 6 hours.

### Evaluation by gas chromatography methods

Samples of (4) are obtained by liquid-liquid extract with dichloromethane and subsequent centrifugation. 1 µL aliquots are analyzed by GC-FID (HP 5890 Series II) and GC-MS (Agilent 5973) using a DB 1701 capillary (Agilent; 30 m x 0.32 mm ID x 1 µm FD). The GC oven is ramped from 40-240 °C at a 5 °C/min heating rate. A Gerstel CIS 3 injector was used.

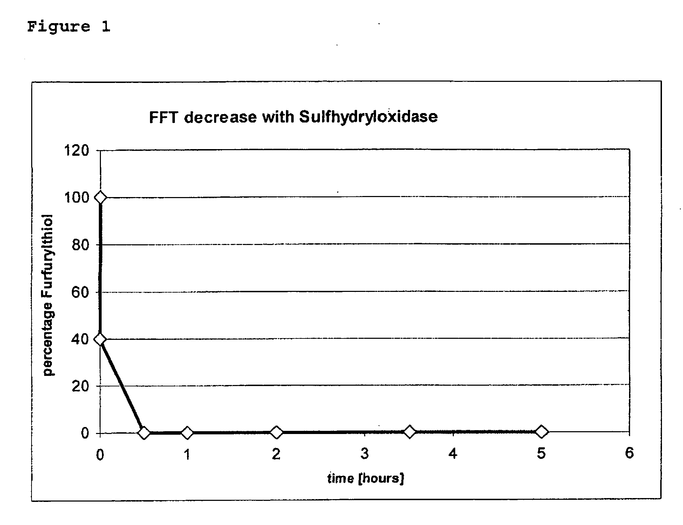

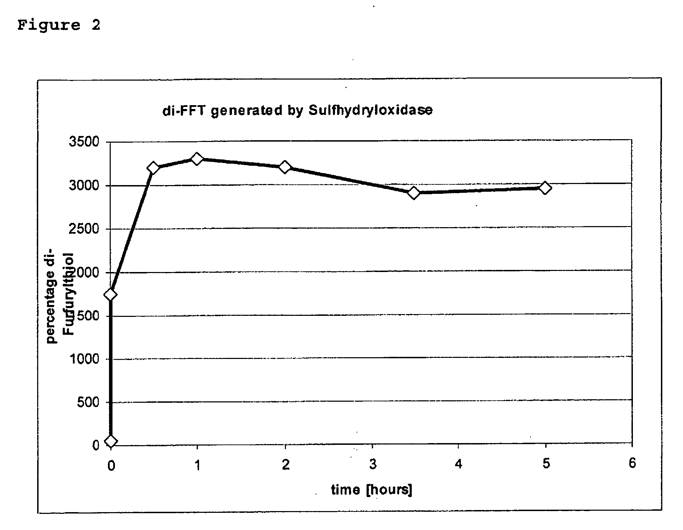

The enzyme-catalyzed decrease of FFT over time and the increase of Di-FFT in the aroma extract are depicted in Figures 1 and 2, respectively.

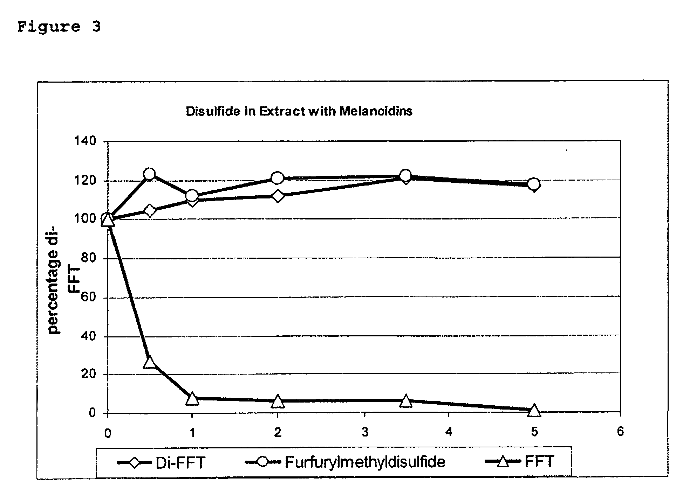

The stability of the resulting 2,2-dithiodimethylendifuran (Di-FFT) is illustrated in Figure 3, compared to methyl-2-methyl-3-furfuryldisulfid and to 2-furfurylthiol.

The aroma impact of Di-FFT (roasty, sulphurous) is comparable to FFT but is milder than thiols. In this respect, 2,2-dithiodimethylendifuran (Di-FFT) in an amount of 10-20 ppb and Methyl-2-methyl-3-furfuryldisulfide in an amount of 10-50 ppb have been spiked into an aged coffee and tasted. Sensory descriptions were fresh, roasty, sulfury, but milder than FFT.

### Example 2

### Preparation of Sulfhydryl Oxidase Solution (5)

100 mg of sulfhydryl oxidase Erv1p from yeast (X-Zyme GmbH, Merowingerplatz 1A, 40225 Düsseldorf, Germany) are dissolved in 2 mL Mcllvaine buffer (1 mM) at pH 7.5.

### Preparation of a flavor-stabilized aroma extract (6)

5 mL of Aroma Extract Solution (1) of Example 1 are supplemented with 2 mL of Sulfhydryl Oxidase Solution (5) and agitated in a 20 mL vial at 750 rpm at 40 °C for 2 ½ hours. Every 30 minutes oxygen is injected into the vial.

### Evaluation by gas chromatography methods

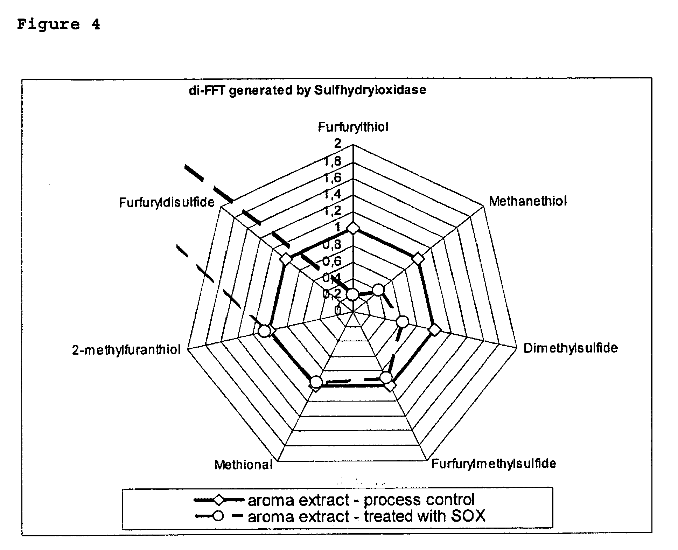

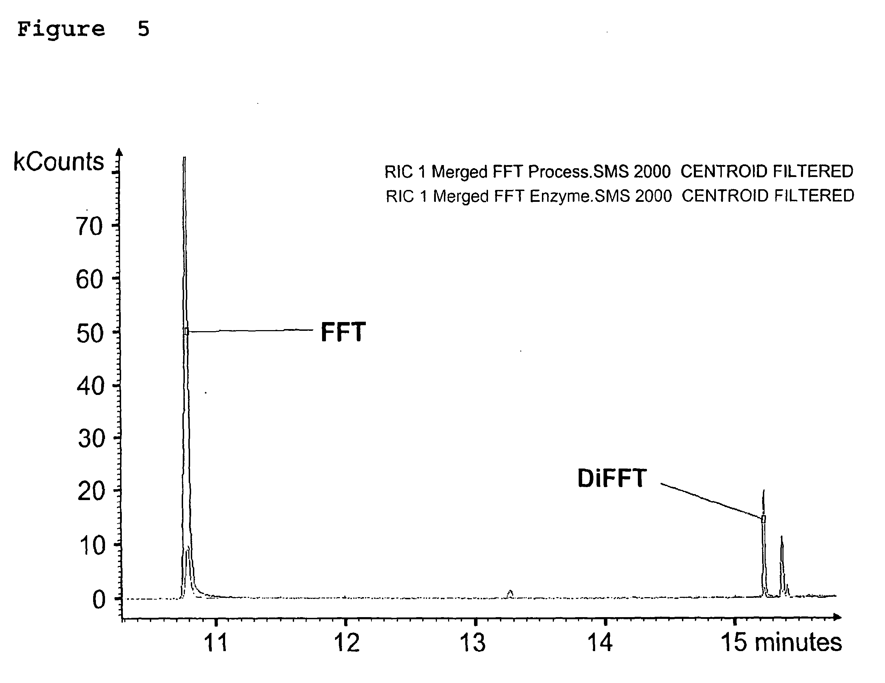

Samples are obtained by liquid-liquid extract of the mixture of (1) and (5) with dichloromethane and subsequent centrifugation. 1 µL aliquots are analyzed by GC-FID (HP 5890 Series II) and GC-MS (Agilent 5973) using a DB 1701 capillary (Agilent; 30 m x 0.32 mm ID x 1 µm FD). The GC oven is ramped from 40-240 °C at a 5 °C/min heating rate. A Gerstel CIS 3 injector was used. The net increase of Di-FFT and a decrease of FFT is observed after 4 hours reaction time (Figure 4). Figure 5 shows a representative GC chromatogram.

### Comparative Example 1

### Preparation of aroma extract (7)

5 mL of Aroma Extract Solution (1) of Example 1 are supplemented with 2 mL of Mcllvaine buffer and agitated in a 20 mL vial at 750 rpm at 40 °C for 2 ½ hours. Every 30 minutes oxygen is injected into the vial.

The reaction has been followed by gas chromatography methods as indicated in Examples 1 and 2. A significant increase of Di-FFT is not observed after 4 hours of reaction time as compared to the enzyme-catalysed reaction (Figure 4).

### Example 3

### Preparation of Sulfhydryl Oxidase Solution (9)

50 mg of sulfhydryl oxidase Erv1p from yeast (X-Zyme GmbH, Merowingerplatz 1A, 40225 Düsseldorf, Germany) are dissolved in 100 mL Mcllvaine buffer (1 mM) at pH 7.54.

### Preparation of Enzyme-Reacted Aroma Extract (10)

150 mL of the Aroma Extract Solution (1) of Example 1 is supplemented with 60 mL of the Sulfhydryl Oxidase Solution (9), oxygen sparging every 30 minutes reaction time and incubated at 40 °C for 5 hours.

### Evaluation of Shelf Life Stability

### Reference Sample 1

5% of the Aroma Extract (8) in a dark Robusta coffee base has been diluted 1/5 in water.

### Sample 2

5% of Enzyme-Reacted Aroma Extract (10) in a dark Robusta coffee base has been diluted 1/5 in water.

Reference Sample 1 and Sample 2 are stored at 50°C for 8 days. Sensory evaluation of both samples is carried out by an expert tasting panel by sniffing at various intervals. Results are depicted Table 3 below.

**Table 3: Results of the Shelf Life Test after 8 days at 50°C**

| | **Flavor** | **average Freshness*** |
|---|---|---|
| Reference Sample 1 | cigar-like, earthy, musty | 2-3 |
| Sample 2 | buttery, fresh, roasty | 3-4 |

| | | |
|---|---|---|
| *Freshness scale: 0-6, wherein 0 indicates no freshness, and 6 indicates high freshness. | | |

As is evident from the Examples, the present method results in disulfides of thiol-containing flavor compound being kept within the mixture, and which have firstly an increased stability in the presence of other coffee components as compared to the unstable thiol compounds and, secondly, which preserve the characteristic flavor. Thus, the present invention provides a selective method of increasing the stability of flavors and aromas without generating unwanted side products.

## Claims

1. A method for increasing the stability of thiol-containing flavor or aroma compounds comprising a step of
(a) contacting or admixing the thiol-containing flavor or aroma with an enzyme catalyzing the formation of disulfides, and
(b) bringing the mixture of (a) into contact with oxygen.

2. The method of claim 1, comprising a step of (c) agitating the mixture.

3. The method of any of claim 1 or 2, comprising the step of supplementing the food with flavors prior to, during and/or after the step (a) of claim 1.

4. The method of any of claims 1-3, wherein the thiol-containing flavor or aroma is a food selected from coffee, coffee mixes, liquid concentrates thereof, tea, cacao, chocolate, peanuts, cheese, cheese flavor blocks, wine and beer.

5. The method of any of claims 1-4, wherein the enzyme catalyzing the formation of disulfides is a sulfhydryl oxidase from yeast.

6. The method of claim 5, wherein the sulfhydryl oxidase is sulfhydryl oxidase Ervlp.

7. A method of any of claims 1-6, wherein the enzyme is immobilized onto or within an insoluble matrix.

8. The method of any of claims 1-7, wherein the oxygen is brought into contact with the mixture by bubbling or by injecting oxygen there through.

9. The method of any of claims 5-8, wherein the molar ratio of sulfhydryl oxidase to thiol groups is from 1:2000 - 2000:1.

10. The method of claim 9, wherein the ratio of sulfhydryl oxidase to thiol groups is 1:1.

11. The method of any of claims 1-10, wherein the catalyzed formation of disulfides is performed in a time range of 5 minutes to 12 hours.

12. The method of claim 11, wherein the time range is from 4-6 hours.

13. A product obtainable by the method according to any of claims 1-12.

14. Product according to claim 13, which is packaged or encapsulated.

## Patentansprüche

1. Verfahren zur Erhöhung der Stabilität von Thiolenthaltenden Geschmacks- oder Aromaverbindungen, das einen Schritt des
(a) In-Kontakt-Bringens oder Beimischens des Thiolenthaltenden Geschmacks- oder Aromastoffs mit einem Enzym, das die Bildung von Disulfiden katalysiert, und
(b) In-Kontakt-Bringens der Mischung aus (a) mit Sauerstoff umfasst.

2. Verfahren gemäß Anspruch 1, das einen Schritt des (c) Rührens der Mischung umfasst.

3. Verfahren gemäß irgendeinem der Ansprüche 1 oder 2, das einen Schritt des Ergänzens von Nahrung mit Geschmacksstoffen vor, während und/oder nach dem Schritt (a) von Anspruch 1 umfasst.

4. Verfahren gemäß irgendeinem der Ansprüche 1 bis 3, worin der Thiol-enthaltende Geschmacks- oder Aromastoff ein Nahrungsmittel ausgewählt aus Kaffee, Kaffeemischungen, flüssige Konzentrate davon, Tee, Kakao, Schokolade, Erdnüsse, Käse, Käsegeschmacks-Blöcke, Wein und Bier ist.

5. Verfahren gemäß irgendeinem der Ansprüche 1 bis 4, worin das Enzym, das die Bildung von Disulfiden katalysiert, eine Sulfhydryloxidase aus Hefe ist.

6. Verfahren gemäß Anspruch 5, worin die Sulfhydryloxidase Sulfhydryloxidase Ervlp ist.

7. Verfahren gemäß irgendeinem der Ansprüche 1 bis 6, worin das Enzym auf oder in einer unlöslichen Matrix immobilisiert ist.

8. Verfahren gemäß irgendeinem der Ansprüche 1 bis 7, worin der Sauerstoff mit der Mischung durch Hindurchblasen oder Injizieren in Kontakt gebracht wird.

9. Verfahren gemäß irgendeinem der Ansprüche 5 bis 8, worin das molare Verhältnis von Sulfhydryloxidase zu Thiolgruppen von 1:2.000 bis 2.000:1 ist.

10. Verfahren gemäß Anspruch 9, worin das Verhältnis von Sulfhydryloxidase zu Thiolgruppen 1:1 ist.

11. Verfahren gemäß irgendeinem der Ansprüche 1 bis 10, worin die katalysierte Disulfidbildung in einem Zeitbereich von 5 Minuten bis 12 Stunden durchgeführt wird.

12. Verfahren gemäß Anspruch 11, worin der Zeitbereich von 4 bis 6 Stunden ist.

13. Erzeugnis, erhältlich durch das Verfahren gemäß irgendeinem der Ansprüche 1 bis 12.

14. Erzeugnis gemäß Anspruch 13, das verpackt oder eingekapselt ist.

## Revendications

1. Procédé pour augmenter la stabilité de composés servant de parfums ou d'arômes contenant des groupes thiol, comprenant une étape
(a) de mise en contact ou de mélange du parfum ou de l'arôme contenant des groupes thiol avec une enzyme catalysant la formation de disulfures, et
(b) de mise du mélange de (a) en contact avec de l'oxygène.

2. Procédé suivant la revendication 1, comprenant une étape
(c) d'agitation du mélange.

3. Procédé suivant l'une quelconque des revendications 1 et 2, comprenant l'étape d'incorporation à la denrée alimentaire de parfums avant, pendant et/ou après l'étape (a) de la revendication 1.

4. Procédé suivant l'une quelconque des revendications 1 à 3, dans lequel le parfum ou l'arôme contenant des groupes thiol est une denrée alimentaire choisie entre le café, des mélanges pour café, des concentrés liquides pour ceux-ci, le thé, le cacao, le chocolat, les cacahuètes, le fromage, des blocs d'arômes de fromage, le vin et la bière.

5. Procédé suivant l'une quelconque des revendications 1 à 4, dans lequel l'enzyme catalysant la formation de disulfures est une sulfhydryloxydase provenant d'une levure.

6. Procédé suivant la revendication 5, dans lequel la sulfhydryloxydase est la sulfhydryloxydase Erv1p.

7. Procédé suivant l'une quelconque des revendications 1 à 6, dans lequel l'enzyme est immobilisée sur ou dans une matrice insoluble.

8. Procédé suivant l'une quelconque des revendications 1 à 7, dans lequel l'oxygène est mis en contact avec le mélange par barbotage ou par injection d'oxygène à travers ce mélange.

9. Procédé suivant l'une quelconque des revendications 5 à 8, dans lequel le rapport molaire de la sulfhydryloxydase aux groupes thiol va de 1:2000 à 2000:1.

10. Procédé suivant la revendication 9, dans lequel le rapport de la sulfhydryloxydase aux groupes thiol est égal à 1:1.

11. Procédé suivant l'une quelconque des revendications 1 à 10, dans lequel la formation catalysée de disulfures est effectuée dans un intervalle de temps de 5 minutes à 12 heures.

12. Procédé suivant la revendication 11, dans lequel l'intervalle de temps va de 4 à 6 heures.

13. Produit pouvant être obtenu par le procédé suivant l'une quelconque des revendications 1 à 12.

14. Produit suivant la revendication 13, qui est emballé ou encapsulé.
